(19) Europäisches Patentamt
European Patent Office
Office européen des brevets

(11) EP 1 998 778 B1

(12) **FASCICULE DE BREVET EUROPEEN**

(45) Date de publication et mention de la délivrance du brevet:
**01.09.2010 Bulletin 2010/35**

(21) Numéro de dépôt: **07731236.1**

(22) Date de dépôt: **30.03.2007**

(51) Int Cl.:
***A61K 31/52*** *(2006.01)* ***A61K 45/06*** *(2006.01)*
***A61P 25/00*** *(2006.01)* ***A61P 25/08*** *(2006.01)*
***A61P 25/14*** *(2006.01)* ***A61P 25/16*** *(2006.01)*
***A61P 25/28*** *(2006.01)* ***A61P 7/04*** *(2006.01)*
***A61P 9/10*** *(2006.01)*

(86) Numéro de dépôt international:
**PCT/FR2007/000558**

(87) Numéro de publication internationale:
**WO 2007/118984 (25.10.2007 Gazette 2007/43)**

(54) **UTILISATION DE LA ( S ) -ROSCOVITINE POUR LA PREVENTION ET/OU LE TRAITEMENT DE MALADIES NEUROLOGIQUES**

VERWENDUNG VON (S)-ROSCOVITIN ZUR VORBEUGUNG UND/ODER BEHANDLUNG VON NEUROLOGISCHEN ERKRANKUNGEN

USE OF (S)-ROSCOVITINE FOR THE PREVENTION AND/OR TREATMENT OF NEUROLOGICAL DISORDERS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **30.03.2006 FR 0602773**

(43) Date de publication de la demande:
**10.12.2008 Bulletin 2008/50**

(73) Titulaire: **Neurokin**
**13009 Marseille (FR)**

(72) Inventeurs:
- **TIMSIT, Serge**
  **29200 Brest (FR)**
- **MENN, Bénédicte**
  **F-13008 Marseille (FR)**
- **MEIJER, Laurent**
  **F-29680 Roscoff (FR)**

(74) Mandataire: **Novagraaf Technologies**
**122 rue Edouard Vaillant**
**92593 Levallois-Perret Cedex (FR)**

(56) Documents cités:
**EP-A- 0 911 634** **EP-A- 1 348 707**
**WO-A-01/70231** **WO-A-97/20842**
**WO-A-2004/026246** **WO-A-2006/021803**
**US-A1- 2003 069 259**

- **MAPELLI MARINA ET AL: "Mechanism of CDK5/p25 binding by CDK inhibitors" JOURNAL OF MEDICINAL CHEMISTRY, vol. 48, no. 3, 10 février 2005 (2005-02-10), pages 671-679, XP002402136 ISSN: 0022-2623**
- **BEART P M ET AL: "Roles for cyclin-dependent and mitogen-activated protein kinases in kainate receptor-mediated apoptosis" SOCIETY FOR NEUROSCIENCE ABSTRACTS, vol. 26, no. 1-2, 2000, pages Abstract No.-702.3, XP008069586 & 30TH ANNUAL MEETING OF THE SOCIETY OF NEUROSCIENCE; NEW ORLEANS, LA, USA; NOVEMBER 04-09, 2000 ISSN: 0190-5295**
- **BACH STEPHANE ET AL: "Roscovitine targets, protein kinases and pyridoxal kinase" JOURNAL OF BIOLOGICAL CHEMISTRY, vol. 280, no. 35, septembre 2005 (2005-09), pages 31208-31219, XP002402137 ISSN: 0021-9258**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne le domaine du traitement et de la prévention de maladies neurologiques notamment associées à des lésions neurologiques liées notamment au phénomène d'excitotoxicité. Elle concerne tout particulièrement une nouvelle application thérapeutique de la S-roscovitine, dont le nom chimique est la 6(benzylamino) 2(S)[[1(hydroxymethyl)propyl]amino]-9-zisopropylpurine).

L'excitotoxicité correspond à une accumulation d'acides aminés excitateurs qui activent excessivement les récepteurs au glutamate conduisant à une mort neuronale (Olney JW and Ishimaru MJ. 1999 ; Excitotoxic cell death. Cell death and diseases of the nervous system. Humana Press Inc :197-219). Les acides aminés excitateurs représentent un groupe d'analogues structuraux du glutamate comprenant de nombreux membres dont l'aspartate, le kainate et certains de ses dérivés, connus pour représenter de puissants excitateurs neuronaux. Le glutamate est incontestablement l'acide aminé excitateur le mieux caractérisé. L'effet des acides aminés excitateurs est transmis par les récepteurs au glutamate métabotropiques et ionotropiques de type NMDA, AMPA et kainate.

L'excitotoxicité joue ainsi un rôle majeur dans le développement de lésions neurologiques associées à de nombreuses maladies neurologiques notamment aigues et chroniques. (Choi, 1988, Trends Neurosci, vol. 11, pages 465-459 ; Coyle and Puttfarcken, 1993, Science, vol. 262, pages 689-695 ; Lipton and Rosenberg, 1994, New Engl J Med, vol. 330, pages 613-622).

Il est donc intéressant d'identifier et de caractériser des composés neuroprotecteurs permettant de prévenir et/ou de traiter les lésions neurologiques notamment liées au phénomène d'excitotoxicité.

L'utilisation de l'isomère R et du mélange racémique de la roscovitine dans le traitement de l'apoptose neuronale a été décrite précédemment dans le brevet Européen EP 0 874 847. Ce brevet met en évidence les propriétés antimitotiques de la roscovitine et notamment son action inhibitrice sur différentes protéines kinases cycline-dépendantes (cdk) impliquées dans le cycle de division cellulaire ou l'apoptose. Sur la base de ces résultats et s'appuyant sur les relations connues entre le cycle de division cellulaire et l'apoptose (Vermeulen et al., Cell Prolif. 2003 vol. 36(3), pages 131-49), les auteurs de ce brevet ont suggéré un effet éventuel de la roscovitine sur l'apoptose neuronale.

L'utilisation de l'isomère R de la roscovitine, en tant qu'inhibiteur sélectif des Cdc2, Cdk2 et Cdk5, dans le traitement de maladies associées à l'apoptose, notamment les maladies neurodégénératives chroniques telles que la maladie d'Alzheimer, de Huntington ou la sclérose latérale amyotrophique, a été précédemment décrite dans la Demande de Brevet européen EP 0 911 634.

L'utilisation de l'isomère R de la roscovitine, en tant qu'inhibiteur de la cdk5, dans le traitement de la maladie d'Alzheimer, a été décrite précédemment dans la Demande Internationale WO 97/20842.

L'utilisation de l'isomère R de la roscovitine, en tant qu'inhibiteur de kinases cyclines dépendantes, dans le traitement de maladies neurodégénératives associées à des lésions neurales aiguës excitotoxiques, notamment l'épilepsie et l'ischémie cérébrale, a été précédemment décrite dans la Demande Internationale WO 01/70231.

L'utilisation de la roscovitine, en tant qu'inhibiteur de CDK2 et/ou CDK7 et/ou CDK9, dans le traitement de maladies associées aux anticorps antinucléaires, notamment les maladies rhumatismales auto-immunes telles que le lupus érythémateux disséminé, a été décrite précédemment dans la Demande Internationale WO 2006/021803.

Bien que certains composés soient déjà utilisés pour traiter les lésions neurologiques, ils peuvent présenter des effets secondaires, comme une certaine toxicité ou une efficacité insuffisante.

Il subsiste donc un besoin pour des composés présentant des propriétés améliorées pour traiter les lésions neurologiques.

De manière surprenante, les inventeurs ont découvert que l'isomère S de la roscovitine permet de résoudre en tout ou partie les problèmes évoqués ci-dessus et présente une meilleure efficacité neuroprotectrice que celle de l'isomère R. Ainsi, les inventeurs ont maintenant identifié et caractérisé un composé particulier, la (S)-roscovitine permettant de prévenir et/ou de traiter efficacement les lésions neurologiques notamment liées au phénomène d'excitotoxicité.

Cet effet neuroprotecteur de la (S)-roscovitine est particulièrement inattendu. En effet, la (S)-roscovitine inhibe plus faiblement différentes protéines kinases cycline-dépendantes (cdk) impliquées dans le cycle de division cellulaire ou l'apoptose que la (R)-roscovitine. Notamment, l'activité inhibitrice de la (S)-roscovitine sur les protéines kinases cdk-5, cdk-1/cyclin B et la cdc2/cyclin B, est moins élevée que celle de la (R)-roscovitine (De Azevedo et al., Eur. J. Biochem. 243, 518-526, 1997 ; Bach et al., The Journal of Biochemical Chemistry, 280, 35, 31208-31219). Or, ces protéines kinases, notamment la cdk-5 et la cdc2/cyclin B, sont connues pour leur rôle dans la mort neuronale (Dhavan and Tsai, 2001 ; Busser et al. 1998).

Selon un premier aspect, l'invention a pour objet l'utilisation de la (S)-roscovitine ou 6-(benzylamino)-2 (*S*)-[[1-(hydroxymethyl)propyl]amino]-9-isopropylpurine) dans un excès énantiomérique supérieur ou égal à 90% ou au moins un de ses sels pharmaceutiquement acceptable pour la fabrication d'un médicament destiné à la prévention et/ou au traitement de maladies neurologiques chroniques choisies dans le groupe constitué par :

- les maladies neurodégénératives ;
- les démences ; et
- les maladies démyélinisantes ;

et les maladies neurologiques aiguës choisies dans le groupe constitué par :

- l'épilepsie ;
- l'état de mal épileptique ;
- l'accident vasculaire cérébral ;
- les hémorragies cérébrales ;
- les hypoxies cérébrales au cours des arrêts cardiaques ;
- les traumatismes crâniens ; et
- les maladies neurologiques entraînant une hypoxie cérébrale focale et/ou globale.

On entend par « (S)-roscovitine », le composé de formule suivante :

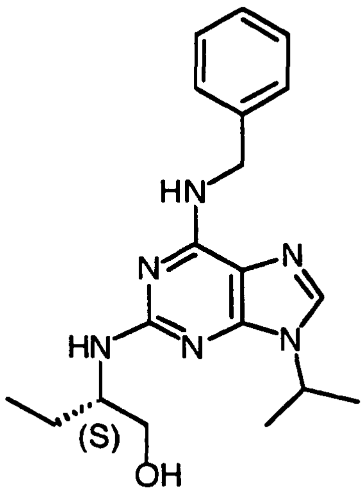

6-(benzyl-amino)-2(*S*)-[[1-(hydroxymethyl) propyl]amino]-9-isopropylpurine) dans un excès énantiomérique supérieur ou égal à 90 %, notamment supérieur ou égal à 95 %, tout particulièrement supérieur ou égal à 99 %, voire supérieur ou égal à 99,5 %.

L'excès énantiomérique peut être défini par la formule ((S)-roscovitine - (R)-roscovitine / (S)-roscovitine + (R)-roscovitine) x 100.

La (S)-roscovitine peut être obtenue selon des techniques bien connues de l'Homme du Métier, par exemple par une synthèse en trois étapes à partir de la 2,6-dichloropurine telle que décrite par Havlicek et al. (J. Med. Chem, 1997, 40, 408) et par Wang et al. (Tetrahefron : Asymmetry, 2001, 12, 2891).

La (S)-roscovitine est également disponible chez Alexis Corporation sous la référence ALX-350-293-M001.

On entend par « sels pharmaceutiquement acceptables », des sels appropriés à une utilisation pharmaceutique. À titre d'exemples de sels pharmaceutiquement acceptables, on peut citer le benzène sulfonate, le bromhydrate, le chlorhydrate, le citrate, l'éthanesulfonate, le fumarate, le gluconate, l'iodate, l'iséthionate, le maléate, le méthanesulfonate, le méthylène-bis-b-oxynaphtoate, le nitrate, l'oxalate, le pamoate, le phosphate, le salicylate, le succinate, le sulfate, le tartrate, le théophyllinacétate et le p-toluènesulfate.

Les sels pharmaceutiquement acceptables de la (S)-roscovitine peuvent être obtenus par des techniques bien connues de l'Homme du Métier.

On entend généralement par « maladie neurologique » des maladies caractérisées par des « lésions neurologiques ». On entend par « lésions neurologiques » une altération structurale du système nerveux dans ses caractères anatomiques et physiologiques. La lésion peut être microscopique ou macroscopique. La lésion peut être d'origine traumatique ou causée par une maladie, particulièrement par les maladies neurologiques aigues ou chroniques. Ces lésions neurologiques peuvent toucher différents types cellulaires, les neurones, les astrocytes, les oligodendrocytes, la microglie et les progéniteurs de ces cellules.

Dans certains cas, les lésions neurologiques sont liées au phénomène d'excitotoxicité.

Tout particulièrement, la prévention et/ou le traitement des lésions neurologiques est lié à l'activité de neuroprotection de la (S)-roscovitine.

On entend par « neuroprotection », la capacité d'un composé à prévenir la mort de cellules neurales saines et/ou malades. On entend par cellules neurales les cellules du système nerveux et notamment du cerveau. Ces cellules neurales peuvent notamment être choisies parmi les neurones, les astrocytes et les oligodendrocytes.

La neuroprotection est particulièrement intéressante dans le cas des maladies neurologiques notamment aigues ou chroniques. En effet, ces maladies peuvent être associées à une dégénérescence des cellules neurales les conduisant à leur mort. Ceci peut donc rendre possible l'utilisation de composés permettant de prévenir et/ou de retarder la mort de ces cellules neurales, ou d'au moins d'une partie de ces cellules neurales, saines et/ou malades.

Par exemple, après un accident vasculaire cérébral certaines cellules neurales meurent immédiatement, ou quasi immédiatement, définissant une zone dite « coeur nécrotique ». Toutefois il existe également une zone dite de « pénombre », jouxtant le coeur nécrotique, dans laquelle les cellules peuvent être progressivement affectées avant d'aboutir à la mort cellulaire.

L'utilisation de certains agents thérapeutiques neuroprotecteurs peut permettre d'éviter l'évolution d'au moins une partie de ces cellules vers une mort neurale.

On entend par « maladies neurologiques chroniques », des maladies neurologiques dont les symptômes peuvent être initialement peu marqués, puis qui peuvent évoluer progressivement et s'aggraver, par exemple sur plusieurs années.

Parmi les maladies neurologiques chroniques, on peut citer :

- les maladies neurodégénératives (Adams et Victor ; Third édition ; McGraw-Hill book company ; 1985) comprenant:
- les maladies avec syndrome extrapyramidal, notamment la maladie de Parkinson, la paralysie supranucléaire progressive (maladies de Steel-Richardson et Olzewski),l'atrophie multisystématisée, et la dégénérescence striatonigrique ;
- les démences, notamment la maladie d'Alzheimer, les démences vasculaires, la maladie à corps de Lewy, les démences fronto-temporales, la dégénérescence cortico-basale, la chorée de Huntington et
- les autres maladies neurodégénératives, notamment la sclérose latérale amyotrophique, la maladie de Creutzfeld-Jakob (Choi, 1988 ; Coyle and Puttfarcken, 1993 ; Lipton and Rosenberg, 1994) ;
- les maladies démyélinisantes, notamment la sclérose en plaques, l'encéphalite allergique aiguë disséminée, la maladie de Devic (neuromyélopathie) et les maladies génétiques avec atteinte de la myéline notamment la maladie de Pelizaeus-Merzbacher.

On entend par « maladies neurologiques aigues » des maladies neurologiques dont les symptômes et les signes cliniques peuvent être initialement très marqués et qui peuvent se stabiliser rapidement, par exemple après quelques jours.

Les maladies neurologiques aigues comprennent :

- l'épilepsie ;
- l'état de mal épileptique ;
- l'accident vasculaire cérébral notamment ischémique ;
- les hémorragies cérébrales ;
- les hypoxies cérébrales au cours des arrêts cardiaques ;
- les traumatismes crâniens et
- les maladies neurologiques entraînant une hypoxie cérébrale focale et/ou globale, survenant notamment au cours des circulations extracorporelles, particulièrement lors d'interventions cardiaques et/ou vasculaires et de la chirurgie des carotides.

On entend par hémorragie cérébrales les hémorragies intraparenchymateuses et les hémorragies méningées. Après hémorragie méningée, il peut survenir une ischémie en rapport avec le vasospasme. La S-Roscovitine pourrait prévenir ou diminuer l'ischémie cérébraleconsécutive à une hémorragie méningée.

Tout particulièrement, certaines hémorragies cérébrales peuvent être liées à l'utilisation d'un agent thrombolytique, notamment l'activateur tissulaire de plasminogène (t-PA). En effet, les agents thrombolytiques utilisés dans les premières heures au cours de l'accident vasculaire cérébral ischémique peuvent provoquer des hémorragies cérébrales. Ces hémorragies cérébrales représentent un effet secondaire majeur de la thrombolyse au cours de l'accident ischémique. La S-roscovitine pourrait donc être intéressante en association avec un agent thrombolytique pour diminuer le risque de survenue d'une hémorragie cérébrale en protégeant la barrière hématoencéphalique. La S-roscovitine pourrait agir comme agent anti-aptoptique contre les cellules endothéliales cérébrales. Les cellules endothéliales cérébrales sont une des composantes majeurs de la barrière hématoencéphalique.

Le médicament selon l'invention, peut comprendre en outre au moins un agent anti-neurodégénératif, en

particulier un agent destiné à lutter et/ou prévenir les maladies: neurologiques chroniques et/ou aigues et plus particulièrement, l'accident vasculaire cérébral ischémique.

On entend par « agent anti-neurodégénératif », un composé permettant de lutter et/ou de prévenir la dégénérescence du système nerveux. À titre d'exemples d'agent anti-neurodégénératif, on peut citer les inhibiteurs de l'acétylcholinestérase comme le donepezil, la sélégiline, la rivastigmine, la galantamine, des antiglutamatergiques comme la memantine et le riluzole. Ainsi, la S-Roscovitine peut être utilisée en association avec un médicament anticholinestérasique (donepezil, rivastigmine, galantamine) ou antiglutamatergique (memantine) dans la maladie d'Alzheimer ou d'autres démences, comme la démence vasculaire, la maladie à corps de Lewy, les démences fronto-temporales, la dégénérescence cortico-basale, la chorée de Huntington, la démence parkinsonienne ... La S-roscovitine pourrait être utilisée en association avec le riluzole dans la sclérose latérale amyotrophique.

La (S)-roscovitine et l'agent antineurodégénératif peuvent être administrés simultanément, séparément ou de manière étalée dans le temps.

La (S)-roscovitine et l'agent anti-neurodégénératif peuvent être présents dans le médicament selon l'invention selon un rapport molaire allant de 10/1 à 1/10.

Le médicament selon l'invention, peut comprendre en outre au moins un agent thrombolytique.

On entend par « agent thrombolytique », une substance capable de lyser des caillots de sang, telle que l'activateur de tissu plasminogène (t-PA), la streptokinase, l'urokinase et la desmoteplase.

La (S)-roscovitine et l'agent thrombolytique peuvent être administrés simultanément, séparément ou de manière étalée dans le temps.

La (S)-roscovitine et l'agent thrombolytique peuvent être présents dans le médicament selon l'invention selon un rapport molaire allant de 100/1 à 1/100.

Les agents thrombolytiques peuvent présenter des effets secondaires, ils peuvent par exemple entraîner des hémorragies cérébrales. L'utilisation de (S)-roscovitine en association avec au moins un agent thrombolytique, notamment le t-PA, peut permettre de diminuer certains de ces effets secondaires, et en particulier le risque d'hémorragie cérébrale.

Le médicament selon l'invention, peut comprendre en outre au moins un agent antiagrégant plaquettaire.

À titre d'exemple d'« agent antiagrégant plaquettaire », on peut citer l'acide acétylsalicylique, le chlorhydrate de ticlopidine, le clopidogrel, le dipyridamole, l'abciximab, le flurbiprofen.

La s-roscovitine peut être utilisée en association avec un agent anti-agrégant plaquettaire dans l'accident vasculaire cérébral ischémique.

La (S)-roscovitine et l'agent antiagrégant plaquettaire peuvent être administrés simultanément, séparément ou de manière étalée dans le temps.

La (S)-roscovitine et l'antiagrégant plaquettaire peuvent être présents dans le médicament selon l'invention selon un rapport molaire allant de 10/1 à 1/10.

Lesdits médicaments selon l'invention sont administrables par différentes voies. À titre d'exemples de voies d'administration utilisables pour les médicaments selon l'invention, on peut citer la voie orale, rectale, cutanée, pulmonaire, nasale, sublinguale, la voie parentérale notamment intradermique, sous-cutanée, intramusculaire, intraveineuse, intra-artérielle, intra-rachidienne, intra-articulaire, intra-pleurale, intra-péritonéale.

En particulier, lorsque les maladies neurologiques sont des maladies neurologiques aigues, les voies d'administrations préférées pour les médicaments selon l'invention sont, la voie intraveineuse, intramusculaire, sublinguale, cutanée, tout préférentiellement la voie intraveineuse et la voie intramusculaire et de manière préférée entre toutes la voie intraveineuse.

En particulier, lorsque les maladies neurologiques sont des maladies neurologiques chroniques, la voie d'administration préférée pour les médicaments selon l'invention est la voie orale.

Les médicaments selon l'invention peuvent être administrés en une ou plusieurs fois ou en libération continue, notamment en perfusion continue.

Les médicaments selon l'invention peuvent se présenter sous différentes formes, en particulier, sous une forme choisie dans le groupe comprenant les comprimés, les gélules, les dragées, les sirops, les suspensions, les solutions, les poudres, les granulés, les émulsions, les microsphères et les solutions injectables, de préférence les comprimés, les solutions injectables, les sprays sublinguaux et les patchs cutanés.

Ces différentes formes peuvent être obtenues par des techniques bien connues de l'Homme du Métier.

Les formulations appropriées à une administration par voie parentérale, les véhicules pharmaceutiquement acceptables appropriés à cette voie d'administration et les techniques de formulation et d'administration correspondantes peuvent être réalisées selon des méthodes bien connues de l'Homme du Métier, en particulier celles décrites dans le manuel Remington's Pharmaceutical Sciences (Mack Publishing Co., Easton, Pa., 20ème édition, 2000).

Selon un autre mode de réalisation particulier, le 6-(benzyl-amino)-2(*S*)-[[1-(hydroxymethyl)propyl]amino]-9-isopropylpurine) ou au moins un de ses sels pharmaceutiquement acceptable est présent dans le médicament en une quantité allant de 50 mg à 5 g par unité de prise, en particulier de 100 mg à 2 g.

Le médicament selon l'invention peut être administré en une ou plusieurs prises par jour, de préférence en 1 à 4 prises par jour.

Avantageusement, la (S)-roscovitine peut être administrée en une quantité allant de 1 à 200 mg/kg par jour.

Avantageusement le médicament comprend une quantité de 6-(benzyl-amino)-2(*S*)-[[1-(hydroxyméthyl)propyl] amino]-9-isopropylpurine) ou d'au moins un de ses sels pharmaceutiquement acceptable allant de 50 mg à 5 g.

Selon un autre mode de réalisation particulier de ladite utilisation selon l'invention, ledit médicament comprend en outre un support pharmaceutiquement acceptable.

On entend par « support pharmaceutiquement acceptable », toute matière qui est appropriée à une utilisation dans un produit pharmaceutique.

À titre d'exemples de support pharmaceutiquement acceptable, on peut citer le lactose, l'amidon, éventuellement modifié, la cellulose, l'hydroxypropyl cellulose, l'hydroxypropylméthyl cellulose, le mannitol, le sorbitol, le xylitol, le dextrose, le sulfate de calcium, le phosphate de calcium, le lactate de calcium, les dextrates, l'inositol, le carbonate de calcium, la glycine, la bentonite, la polyvinylpyrriolidone, et leurs mélanges.

Le médicament selon l'invention peut comprendre une teneur en support pharmaceutiquement acceptable allant de 5 à 99 % en poids, notamment de 10 à 90 % en poids, et en particulier de 20 à 75 % en poids par rapport au poids total de la composition.

D'autres avantages et caractéristiques de l'invention apparaîtront au regard des figures et des exemples qui suivent.

Les figures et les exemples qui suivent sont donnés à titre illustratif.

- La Figure 1 illustre sous la forme d'un histogramme l'effet neuroprotecteur de la (S)-roscovitine dans un modèle *in vitro* d'excitotoxicité : une culture neurale mixte (astrocytes, neurones, oligodendrocytes) de cellules de l'hippocampe exposée au kainate.(**$p<0,05$ ; *$p<0,01$ avec un t-test de Student).
- La Figure 2 illustre sous la forme d'un graphique la concentration de neuroprotection (CN50) de la (S)-roscovitine dans une culture neurale mixte de cellules de l'hippocampe exposée au kainate.
- La Figure 3 illustre sous la forme d'un histogramme l'effet de la (S)-roscovitine à différents temps d'incubation sur une culture neurale mixte de cellules de l'hippocampe exposée au kainate. (**$p<0,05$ ; *$p<0,01$ avec un t-test de Student).
- La Figure 4 (A et B) illustre l'effet neuroprotecteur de la (S)-roscovitine dans un modèle *in vitro* complexe d'excitotoxicité : une culture de tranches organotypiques d'hippocampe de rat. (A) Observation de la mort, cellulaire dans la région CA3 d'hippocampe de rat après marquage à l'iodure de propidium et incubation avec soit du DMSO et $H_2O$ (contrôle), soit du kainate, soit du kainate et de la (S)-roscovitine. (B) Représentation sous forme d'histogramme de la mort neuronale relative (MNR) dans la région CA3 d'hippocampe de rat après marquage à l'iodure de propidium et incubation avec soit du DMSO et $H_2O$ (contrôle), soit du kainate, soit du kainate et de la (S)-roscovitine. (*$p<0,01$ avec un t-test de Student).
- La Figure 5 (A et B) illustre la caractérisation des régions de « coeur nécrotique » et de « zone de pénombre » dans des cerveaux de souris dans un modèle *in vivo* d'ischémie focale permanente. (A) Photographie de coupes coronales d'un cerveau de souris adulte colorées par le 2, 3, 5 chlorure de triphenyl tetrazolium (TTC), 3 heures après MCAo. Trois régions coronales peuvent être identifiées et délimitées en fonction de leur intensité de coloration : « le coeur nécrotique », « la zone de pénombre » et le tissu sain. (B) Mesure à l'aide du logiciel ImageJ des intensités relatives de coloration du « coeur nécrotique », de « la zone de pénombre » et du tissu sain colorés par le 2, 3, 5 TTC, 3 heures après MCAo. (*$p<0,01$ avec un t-test de Student).
- La Figure 6 (A et B) illustre sous la forme d'histogrammes l'effet neuroprotecteur de la (S)-roscovitine dans un modèle *in vivo* murin d'ischémie focale permanente. La (S)-roscovitine a été administrée par voie intracérébroventriculaire (IVC) (A) ou par voie systémique (IP) (B). La mort cellulaire a été évaluée par la mesure des intensités relatives de coloration dans le coeur nécrotique, la zone de pénombre. (*$p<0,01$ avec un t-test de Student).
- La Figure 7 illustre la comparaison de l'indice de neuroprotection (IN) de la (S)-roscovitine et de la (R)-roscovitine dans un modèle *in vitro* d'excitotoxicité : une culture neurale mixte de cellules de l'hippocampe exposée au kainate.
- La Figure 8 illustre la mort neuronale sélective induite par le kainate à partir de cultures mixtes d'hippocampes :

  (a-c) : Des cellules d'hippocampes isolées d'embryon de rat E18 et cultivées pendant 10 et 15 jours ont été caractérisées par immunocytochimie avec anticorps spécifiques des différents types cellulaires et enregistrement patch-clamp.

  (a) Photographie au microscope en contraste de phase de cellules maintenues en culture pendant 10 jours.
  (b) Photographie au microscopie confocale à fluorescence de cellules maintenues en culture pendant 10 jours et marquées avec les anticorps anti-GFAP (rouge), anti-bêta-tubuline de classe III (vert) et anti-04 (bleu). Les cultures d'hippocampes contiennent à la fois des cellules neuronales et gliales.

(c) Tracé montrant les enregistrements en voltage-clamp dans une configuration cellule entière de neurones maintenus en culture pendant 10 jours (tracé du haut) et 15 jours (tracé du bas)

(d) Un modèle d'excitotoxicité neuronale a été développé à l'aide de cultures mixtes d'hippocampe de 10 jours soumis à un traitement par kainate. Photographie au microscope à fluorescence de cultures en condition contrôle (à gauche) ou traitées par 200 μM de kainate (à droite) et immunomarquées par l'anticorps anti-bêta-tubuline de classe III (en haut) ou par le marqueur de mort cellulaire, l'iodure de propidium (PI ; en bas). Noter la diminution de la densité cellulaire caractérisée par la bêta-tubuline et l'augmentation de cellules marquées par l'iodure de propidium dans les cultures traitées par le kainate en comparaison avec les cultures contrôles.

(e) Pourcentage relatif de cellules exprimant la bêta-tubuline de classe III dans les cultures contrôlés ou traitées par le kainate.

(f) Excitotoxicité neuronale dose-dépendante du kainate. Dans nos conditions, le traitement par 200 μM de kainate pendant 5 heures est nécessaire pour obtenir environ 50% de mort neuronale ($p< 0, 01$, t-test)

## EXEMPLES

### I. Etude de l'effet neuroprotecteur de la (S)-roscovitine sur la mort neuronale

#### I.1. Étude de l'effet neuroprotecteur de la (S)-roscovitine sur un modèle *in vitro* d'excitotoxicité : une culture neurale mixte de cellules de l'hippocampe

Ce modèle correspond à une culture mixte de cellules neuronales et gliales prélevées à partir d'hippocampe de rats âgés de 18 jours embryonnaires (E18) et exposée au kainate (KA), un analogue du glutamate. Ce système de culture mixte a été préféré à un système de culture exclusive de neurones afin de mieux refléter l'environnement des cellules *in vivo.* Dans ces conditions de culture, les astrocytes et les oligodendrocytes n'ont pas été affectés par le traitement au kainate. La figure 8 illustre la mort neuronale qui a pu ainsi être observée dans un modèle cellulaire complexe d'excitotoxicité strictement neuronale.

Le kainate, un agoniste glutamatergique a été choisi *in vitro* comme agent excitotoxique dans les présentes expérimentations. Ce choix a été fondé entre autres sur des études *in vivo* qui ont monté que le kainate induit une mort cellulaire programmée en comparaison à des agonistes NMDA qui induisent une mort de type nécrotique (Portera-Cailliau ; 1997). Cette mort programmée est aussi visible dans les maladies neurologiques aigues et chroniques. L'importance et la relevance du glutamate ont été récemment soulignées par le fait que des médicaments anti-glutamatergiques commercialisées sont actuellement utilisés chez l'Homme dans la maladie d'Alzheimer (mémantine, Reisberg 2003 ; N. Eng. J. Med ; 348 :1333-1341) et la Sclérose latérale amyotrophique (Riluzole).

I.1.1 Protocole expérimental

Des cultures de cellules d'hippocampe ont été préparées à partir de rats Wïstar âgés de 18 jours embryonnaires (E18) tels que décrits dans Medina et al. (1994, J. Neurophysiol., 72, 456-465). Après 10 jours de culture *in vitro,* les cellules ont été incubées en présence de kainate et/ou de (S)-roscovitine. Les cultures ont été exposées pendant 5 heures à une concentration de 200 μM de kainate. Ces conditions permettent d'obtenir la mort de 40 % à 50 % des neurones en culture. La (S)-roscovitine a été testée à cinq concentrations différentes (0,05 μM; 0,1 μM; 0,5 μM; 1 μM et 5 μM), seule ou en combinaison avec le kainate. La (S)-roscovitine a été ajoutée aux cellules en culture soit simultanément avec le kainate soit à différents temps avant (1 heure) ou après (1, 2, ou 3 heures) l'ajout du kainate.

Les cellules en culture ont été incubées pendant 5 heures avec du kainate et/ou les composés à tester avant l'observation de la mort neuronale. Les témoins ont été incubés uniquement avec les véhicules DMSO et $H_2O$.

La mort neuronale a été évaluée par observation en microscopie à contraste de phase et par l'utilisation de l'iodure de propidium (IP) qui est un marqueur de mort cellulaire. L'iodure de propidium est un marqueur rouge, qui se lie spécifiquement aux acides nucléiques des cellules mortes. Les neurones de champs représentatifs ont été dénombrés. Au moins 5 champs par condition (le nombre total de neurones étant d'environ 150) ont été examinés à partir de 3 cultures indépendantes.

Pour chaque condition expérimentale, le pourcentage de mort neuronale a été exprimé par le rapport entre le nombre de neurones marqués par l'iodure de propidium et le nombre total de neurones visualisés par microscopie à contraste de phase.

Afin de déterminer l'effet neuroprotecteur des composés testés, la mort neuronale relative a été calculée (MNR) et l'indice de neuroprotection (IN) a été défini comme suit :

MNR = % mort neuronale (KA + composés à tester) - % mort neuronale (témoin)/% mort neuronale (KA) - % mort neuronale (témoin) et

IN = 100% – MNR.

Par définition, le pourcentage de mort neuronale relatif (MNR) dans les cellules traitées par le kainate seul était de 100% et l'indice de neuroprotection (IN) était de 0.

La concentration de composés testés nécessaire pour obtenir un indice de neuroprotection (IN) de 50% a été désignée par CN50 : concentration de neuroprotection.

I.1.2 Résultats

I.1.2.1 Effet neuroprotecteur de la S-roscovitine

L'effet de la (S)-roscovitine sur la mort neuronale, présenté sur la Figure 1, a été évalué lorsque la (S)-roscovitine est ajoutée au milieu de culture en même temps que le kainate. Tandis que le pourcentage de mort neuronale (MNR) était de 100% dans le groupe traité par le kainate, celui-ci était respectivement de 81,5%, 50,8%, 27,9%, 21,6% et 15,3% en présence de 0,05 μM 0,1 μM 0,5 μM 1 μM et 5 μM de S-roscovitine.

L'indice de neuroprotection (IN) tel que défini précédemment était respectivement de 18,5%, 49,2%, 72,1%, 78,4% et 84,7% pour des doses de S-roscovitine de 0,05 μM, 0,1 μM, 0,5 μM, 1 μM, et 5 μM.

L'effet neuroprotecteur de la (S)-roscovitine est donc dose-dépendant.

La concentration de neuroprotection telle que définie précédemment a été déterminée à 0,19 μM pour la (S)-roscovitine (Figure 2).

I.1.2.2 Détermination de la fenêtre thérapeutique de l'effet neuroprotecteur de la (S)-roscovitine

La fenêtre thérapeutique de l'effet neuroprotecteur de la (S)-roscovitine, présentée sur la figure 3, a été déterminée en mesurant sa capacité à protéger les neurones lorsque le composé a été ajouté au milieu de culture soit en même temps, soit à différents temps avant (1 heure, T-1) ou après (1 heure, 2 heures ou 3 heures ; T+1, T+2, T+3) l'addition de kainate. Différentes concentrations de (S)-roscovitine ont été étudiées (0,5 μM, 1 μM et 5 μM). Tandis que le pourcentage de mort neuronale (MNR) était de 100% dans les groupes traités par le kainate aux différents temps testés, celui-ci était respectivement de 23.3%, -10.4%, - 14.4% a T-1, 27.9%, 21.6%, 15.3% a T0, 64.9%, 30.7%, 19.5% a T+1, 66.4%, 44.8%, 14.7% a T+2 et de 71.0%, 71.7%, 65.3% a T+3 en présence de 0,5 μM 1 μM et 5 μM de (S)-roscovitine.

L'indice de neuroprotection (IN) tel que défini précédemment était respectivement de 76.6%, 110.4%, 114.4% a T-1, 72.1%, 78.4%, 84.7% a T0, 35.1%, 69.3%, 80.5% a T+1, 33.6%, 55.2%, 85.3% a T+2, et de 29.0%, 28.3%, 34.7% a T+3 pour des doses de (S)-roscovitine de 0,5 μM, 1 μM, et 5 μM.

L'effet neuroprotecteur de la (S)-roscovitine est observé lorsque le composé est ajouté aux cultures jusqu'à 2 heures après l'agent toxique. De plus, l'effet de la (S)-roscovitine est dose-dépendant. Par ailleurs, la (S)-roscovitine a un effet préventif sur la mort neuronale induite par le kainate.

**I.2** Étude de l'effet neuroprotecteur de la (S)-roscovitine dans un modèle *in vitro* complexe d'excitotoxicité : une culture organotypique d'hippocampe de rat

Les cultures organotypiques sont des explants d'organes mis en culture. Ces cultures ont l'avantage d'associer le contrôle des conditions *in vitro* avec la complexité du tissu qui se rapproche de l'environnement in situ. En effet, l'architecture organotypique du tissu nerveux est maintenue dans ces cultures (Stoppini et al., 1991, J Neurosci Methods, vol. 37, pages 173-182). Les cultures s'étalent considérablement, mais restent tridimensionnelles et la morphologie typique des neurones pyramidaux est conservée. L'organisation synaptique et les parcours des fibres hippocampiques intrinsèques se développent d'une manière similaire à la situation *in vivo.* De même, les processus de maturation et de formation de synapses en cultures reflètent ceux décrits *in vivo* (Muller et al., 1993, Dev Brain Res, vol. 71, pages 93-100; Buchs et al., 1993, Dev Brain Res, vol. 71, pages 81-91).

I.2.1 Protocole expérimental

Les cultures organotypiques ont été réalisées à partir d'hippocampes de rats âgés de 2 jours (P2) en utilisant la méthode de Stoppini et al. (1991, J Neurosci Methods, vol. 37, pages 173-182) . Les rats sont sacrifiés par décapitation. Le cerveau est disséqué dans du milieu de dissection (PBS IX, glucose 5.85 g/l) à 4 °C. Des coupes transversales d'une épaisseur de 400 μm sont réalisées à l'aide d'un «tissue chopper» (Mc Ilwain). Une fois séparées les tranches sont mises en culture sur des inserts à membranes poreuses (0.4μm) et transparentes (de 30mm de diamètre), dans du milieu de culture (MEM 1X, 20 % sérum de cheval, insuline 1mg/l). L'intégralité du milieu de culture est remplacée tous les deux jours. Les cultures sont maintenues à 37 °C dans un incubateur où l'atmosphère est enrichie en $CO_2$ (5 %) et humide.

Après 17 jours en culture, le milieu de culture contenant du sérum est remplacé par du milieu frais dépourvu de sérum et en présence d'iodure de propidium (IP ; 7.5μM). 24 heures après l'addition de IP, le milieu est remplacé par du milieu frais dépourvu de sérum contenant du IP et du kainate (5μM) et/ou de la (S)-roscovitine (20μM). Les témoins ont été incubés uniquement avec les véhicules (DMSO et H20). Les cultures sont fixées au bout de 24 heures par une solution de paraformaldehyde 4%.

La mort cellulaire est quantifiée à l'aide du marquage à l'iodure du propidium avec le logiciel ImageJ (NIH). L'intensité du PI est mesurée dans la région CA3 pour chaque condition de traitement.

L'effet neuroprotecteur est examiné en déterminant la mort neuronale relative comme paramètre (MNR) tel que défini dans le paragraphe précédent (I.1.1.).

I.2.2 Résultats

L'intensité de fluorescence du PI est très fortement diminuée dans la région CA3 des cultures traitées à la fois par le Kainate et la (S)-roscovitine par rapport à celles traitées uniquement par le Kainate (Figure 4a). La mort cellulaire induite par le Kainate a également été quantifiée à l'aide du logiciel ImageJ. Nos résultats ont montré que le MNR est de 30.7% en présence de KA/(S)-roscovitine, alors qu'il est arbitrairement de 100% en présence de KA uniquement (Figure 4b).

Ces résultats ont montré d'une part l'absence d'effet toxique de la (S)-roscovitine sur les cultures organotypiques d'hippocampe de rat et d'autre part l'effet neuroprotecteur de la (S)-roscovitine sur la mort neuronale.

**I. 3. Étude de l'effet neuroprotecteur de la (S)-roscovitine sur un modèle *in vivo* d'ischémie: un modèle d'ischémie focale permanente chez la souris.**

Ce modèle consiste en l'occlusion unilatérale par électrocoagulation de l'artère cérébrale moyenne chez l'animal adulte (MCAo ; méthode modifiée de Tamura et al., 1981, J Cereb Blood Flow Metab, vol. 1, pages 53-60). Chez la souris, ce modèle entraîne une atteinte quasi-exclusive du cortex temporo-pariétal de l'hémisphère ipsi-latéral. Ces lésions sont visibles des 3 heures après MCAo et leur taille s'étend avec le temps pour atteindre un maximum à 24 heures (Guegan et al., 1998, Exp Neurol, vol. 154, pages 371-380). À ce stade, la plupart des cellules localisées dans les régions ischémiées présentent les caractéristiques morphologiques et biochimiques de cellules apoptotiques (Guegan et al., 1998, Exp Neurol, vol. 154, pages 371-380 ; Guegan et al., 1998, Mol Brain Res, vol. 55, pages 133-140).

I.3.1. Protocole expérimental

Les ischémies ont été réalisées sur des souris C57b/6 mâles âgées de 60 jours et pesant entre 20-25 g selon le protocole modifié de Tamura et al(1981, J Cereb Blood Flow Metab, vol. 1, pages 53-60) (Guegan et al., 1998, Exp Neurol, vol. 154, pages 371-380). Les animaux ont été anesthésiés par de l'hydrate de chloral (500mg/kg). L'artère cérébrale moyenne (ACM) a été chirurgicalement exposée puis électrocoagulée à l'aide d'une pince bipolaire. La température corporelle des animaux a été maintenue à 37°C pendant toute la chirurgie. Les animaux ont été sacrifiés par dislocation cervicale 3 heures après occlusion de l'ACM.

La (S)-roscovitine a été administrée selon deux modes : intracérébroventriculaire et systémique. Pour la voie intracérébroventriculaire (ICV), la (S)-roscovitine a été administrée à la concentration de 500 μM dans une solution de Kreb's Ringer à l'aide d'une micropompe osmotique (Alzet) implantée 48 heures avant l'occlusion de la MCA dans le ventricule latéral droit de l'animal aux coordonnées stéréotaxiques suivantes : antéro-postérieur = 0, latéral = -0.8, profondeur = 2 (par rapport au Bregma). Pour la voie systémique (IP), la (S)_roscovitine a été administrée à la concentration de 25 mg/kg dans une solution de HCl 0.05M en réalisant 2 injections intrapéritonéales 15 minutes avant et 1 heure après l'occlusion de la MCA. Les animaux témoins ont reçu uniquement les véhicules (DMSO 1% pour l'ICV et HCl 0.05M pour l'IP).

Le volume des lésions cérébrales a été estimé grâce à une coloration par le 2, 3, 5 chlorure de triphenyl

tetrazolium (TTC). Cette coloration se base sur le bon fonctionnement des enzymes mitochondriales. L'intensité de coloration reflète le nombre de mitochondries fonctionnelles. Cette coloration permet ainsi de différencier les régions lésées des régions saines. Les animaux ont été sacrifiés par dislocation cervicale 3 heures après l'occlusion de la MCA. Les cerveaux ont été disséqués et sectionnés en tranches coronales de 1mm d'épaisseur. Les tranches ont ensuite été colorées par une solution de 1% TTC pendant 10 minutes et analysées à l'aide du logiciel NIH ImageJ. Trois régions ont pu être déterminées à 3 heures basées sur l'intensité de la coloration TTC : un coeur nécrotique incolore, une zone de pénombre légèrement colorée et un tissu sain fortement coloré (figure 5). Les volumes du coeur nécrotique, de la zone de pénombre et la lésion totale (coeur+pénombre) ont ainsi été déterminés

I.3.2- Résultats

Les résultats sont présentés sur la Figure 6. L'administration de la (S)-roscovitine par voie intracérébroventriculaire (ICV) a entraîné une réduction de 27.7% du volume total de la lésion 3 heures après l'occlusion de la MCA par rapport au contrôle (18.74 $mm^3$ pour le groupe contrôle et 13.54 $mm^3$ pour le groupe ayant reçu la (S)-roscovitine). Alors que le volume du coeur nécrotique reste inchangé entre les 2 groupes (6.06 $mm^3$ pour le groupe contrôle et 5.39 $mm^3$ pour le groupe ayant reçu la (S)-roscovitine), une forte réduction (35.8 %) de la taille de la zone de pénombre a été observée pour le groupe ayant reçu la (S)-roscovitine par rapport au groupe contrôle (12.68 $mm^3$ pour le groupe contrôle et 8.14 $mm^3$ pour le groupe ayant reçu la (S)-roscovitine) (Figure 6A).

L'administration de la (S)-roscovitine par voie systémique (IP) a entraîné une réduction de 30.7% du volume total de la lésion 3 heures après l'occlusion de la MCA par rapport au contrôle (20.34 $mm^3$ pour le groupe contrôle et 14.10 $mm^3$ pour le groupe ayant reçu la (S)-roscovitine). Alors que le volume du coeur nécrotique reste inchangé entre les 2 groupes (5.03 $mm^3$ pour le groupe contrôle et 4.88 $mm^3$ pour le groupe ayant reçu la (S)-roscovitine), une forte réduction (38.9%) de la taille de la pénombre a été observée pour le groupe ayant reçu la (S)-roscovitine par rapport au groupe contrôle (15.31 $mm^3$ pour le groupe contrôle et 9.22 $mm^3$ pour le groupe ayant reçu la (S)-roscovitine) (Figure 6B).

Ces résultats ont montré que la (S)-roscovitine a un effet neuroprotecteur sur le volume de la lésion dans un modèle sévère d'ischémie focale permanente chez la souris. La (S)-roscovitine agit sur le volume de la zone de pénombre et non sur le coeur nécrotique de la lésion. Par ailleurs ces résultats montrent que la (S)-roscovitine est efficace après une administration systémique du composé, suggérant que la (S)-roscovitine est capable de traverser la barrière hématoencéphalique.

II Comparaison de l'effet neuroprotecteur de la (S)-roscovitine et de la (R)-roscovitine

L'effet de la (S)-roscovitine sur la mort neuronale a été comparé à celui de la (R)-roscovitine dans le système d'étude in vitro tel que défini précédemment en I.1.

Le pourcentage de mort neuronale (MNR) en présence de la (R)-roscovitine est présenté sur la Figure 7.

La (S)-roscovitine à une concentration de 0,5 $\mu$M permet d'obtenir un MNR de 27,9 % alors qu'une concentration de 0,5 $\mu$M de (R)-roscovitine permet d'obtenir un MNR de 62 %. Ainsi dans ces conditions, la (S)-roscovitine permet de prévenir la mort de 2 fois plus de neurones que la R-roscovitine.

La concentration de neuroprotection (CN50) de la (R)-roscovitine est de 0,65 $\mu$M tandis qu'elle est de 0,19 $\mu$M pour la (S)-roscovitine. Il faut donc plus de trois fois plus de (R)-roscovitine que de (S)-roscovitine pour prévenir la mort d'un même nombre de neurones.

Ces expériences montrent bien que l'effet neuroprotecteur de la (S)-roscovitine est plus important que celui de la (R)-roscovitine.

## Revendications

1. Utilisation du 6-(benzyl-amino)-2(*S*)-[[1-(hydroxyméthyl)propyl]amino]-9-isopropylpurine) dans un excès énantiomérique supérieur ou égal à 90% ou au moins un de ses sels pharmaceutiquement acceptables pour la fabrication d'un médicament destiné à la prévention et/ou au traitement de maladies neurologiques chroniques choisies dans le groupe constitué par :

   - les maladies neurodégénératives :
   - les démences, et
   - les maladies démyélinisantes ;

   et les maladies neurologiques aigues choisies dans le groupe constitué par :

- l'épilepsie ;
- l'état de mal épileptique ;
- l'accident vasculaire cérébral ;
- les hémorragies cérébrales ;
- les hypoxies cérébrales au cours des arrêts cardiaques ;
- les traumatismes crâniens et
- les maladies neurologiques entraînant une hypoxie cérébrale focale et/ou globale.

2. Utilisation selon la revendication 1 **caractérisée en ce que** lesdites maladies neurodégénératives sont choisies dans le groupe constitué par les maladies avec syndrome extrapyramidal, la sclérose latérale amyotrophique et la maladie de Creutzfeld-Jocob.

3. Utilisation selon la revendication 2 **caractérisée en ce que** lesdites maladies avec syndrome extrapyramidal sont choisies dans le groupe constitué par la maladie de Parkinson, la paralysie supranucléaire progressive notamment les maladies de Steel-Richardson et Olzewski, l'atrophie multisystématisée, et la dégénérescence striato-nigrique.

4. Utilisation selon la revendication 1 **caractérisée en ce que** lesdites démences sont choisies dans le groupe constitué par la maladie d'Alzheimer, les démences vasculaires, la maladie à corps de Lewy, les démences fronto-temporales, la dégénérescence cortico-basale, la chorée de Huntington.

5. Utilisation selon la revendication 1 **caractérisée en ce que** lesdites maladies démyélinisantes sont choisies dans le groupe constitué par la sclérose en plaques, l'encéphalite allergique aiguë disséminée, la maladie de Devic et les maladies génétiques avec atteinte de la myéline.

6. Utilisation selon la revendication 1, **caractérisée en ce que** les hémorragies cérébrales sont liées à l'utilisation d'un agent thrombolytique.

7. Utilisation selon la revendication 6, **caractérisée en ce que** l'agent thrombolytique est l'activateur tissulaire du plasminogène.

8. Utilisation selon l'une quelconque des revendications 1 à 7, **caractérisée en ce que** ledit médicament comprend en outre au moins un agent anti-neurodégénératif choisi dans le groupe comprenant le donepezil, la sélégiline, la rivastigmine, la galantamine, la memantine et le riluzole.

9. Utilisation selon l'une quelconque des revendications 1 à 8, **caractérisée en ce que** ledit médicament comprend en outre au moins un agent thrombolytique choisi dans le groupe comprenant l'activateur de tissu plasminogène, la streptokinase, l'urokinase et la desmoteplase.

10. Utilisation selon l'une quelconque des revendications 1 à 9, **caractérisée en ce que** ledit médicament comprend en outre au moins un agent antiagrégant plaquettaire choisi dans le groupe comprenant l'aspirine, la ticlopidine, le clopidogrel, la persantine, l'abciximab et le flurbiprofen.

11. Utilisation selon l'une quelconque des revendications 1 à 10, **caractérisée en ce** ledit médicament est administrable par la voie orale, rectale, cutanée, pulmonaire, nasale, sublinguale, la voie parentérale notamment intradermique, sous-cutanée, intramusculaire, intraveineuse, intra-artérielle, intra-rachidienne, intra-articulaire, intra-pleurale, intra-péritonéale.

12. Utilisation selon l'une quelconque des revendications 1 à 11, **caractérisée en ce que** ledit médicament se présente sous une forme choisie dans le groupe comprenant les comprimés, les gélules, les dragées, les sirops, les suspensions, les solutions, les poudres, les granulés, les émulsions, les microsphères, les solutions injectables, les sprays sublinguaux et les patchs cutanés.

13. Utilisation selon l'une quelconque des revendications 1 à 12, **caractérisée en ce que** ledit 6-(benzyl-amino)-2(*S*)-[[1-(hydroxymethyl)propyl]amino]-9-isopropylpurine) ou un de ses sels pharmaceutiquement acceptable est présent dans ledit médicament en une quantité allant de 100 mg à 5 g par unité de prise, de préférence de 100 mg à 2 g.

14. Utilisation selon l'une quelconque des revendications 1 à 13, **caractérisée en ce que** ledit médicament comprend en outre un support pharmaceutiquement acceptable.

## Claims

1. Use of 6-(benzyl-amino)-2(*S*)-[[1-(hydroxymethyl)propyl]amino]-9-isopropylpurine) in an enantiomeric excess greater than or equal to 90% or at least one of its pharmaceutically acceptable salts for manufacturing a medication intended for the prevention and/or treatment of chronic neurological diseases chosen from the group consisting of :

   - neurodegenerative diseases ;
   - dementias ; and
   - demyelinating diseases ;

   and acute neurological diseases chosen from the group consisting of :

   - epilepsy ;
   - status epilepticus ;
   - stroke;
   - cerebral haemorrhages ;
   - cerebral hypoxia during cardiac arrests ;
   - cranial traumatisms ; and
   - neurological diseases causing focal and/or global cerebral hypoxia.

2. Use according to claim 1, wherein said neurodegenerative diseases are chosen from the group consisting of diseases with extrapyramidal syndrome, amyotrophic lateral sclerosis and Creutzfeld-Jakob disease.

3. Use according to claim 2, wherein said diseases with extrapyramidal syndrome are chosen from the group consisting of Parkinson's disease, progressive supranuclear paralysis in particular Steel-Richardson and Olzewski syndromes, multiple system atrophy, and striatonigral degeneration.

4. Use according to claim 1, wherein said dementias are chosen from the group consisting of Alzheimer's disease, vascular dementias, Lewy body disease, frontotemporal dementias, cortico-basal degeneration, and Huntington's chorea.

5. Use according to claim 1, wherein said demyelinating diseases are chosen from the group consisting of multiple sclerosis, disseminated acute allergic encephalitis, Devic's disease, and genetic diseases with affliction of the myelin.

6. Use according to claim 1, wherein cerebral haemorrhages are linked to the use of a thrombolytic agent.

7. Use according to claim 6, wherein the thrombolytic agent is the tissue plasminogen activator.

8. Use according to any one of claims 1 to 7, wherein said medication further comprises at least one anti-neurodegenerative agent chosen from the group comprising donepezil, selegiline, rivastigmine, galantamine, memantine, and riluzole.

9. Use according to any one of claims 1 to 8, wherein said medication further comprises at least one thrombolytic agent chosen from the group comprising the tissue plasminogen activator, streptokinase, urokinase, and desmoteplase.

10. Use according to any one of claims 1 to 9, wherein said medication further comprises at least one platelet aggregation inhibiting agent chosen from the group comprising aspirin, ticlopidine, clopidogrel, persantine, abciximab, and flurbiprofen.

11. Use according to any one of claims 1 to 10, wherein said medication can be administered by oral, rectal, cutaneous, pulmonary, nasal, sublingual, parenteral administration in particular intradermic, subcutaneous, intramuscular, intraveneous, intraarterial, intrarachidian, intra-articular, intrapleural, intraperitoneal administration.

12. Use according to any one of claims 1 to 11, wherein said medication is in a form chosen from the group comprising tablets, capsules, pills, syrups, suspensions, solutions, powders, granules, emulsions, microspheres, injectable solutions, sublingual sprays, and skin patches.

13. Use according to any one of claims 1 to 12, wherein said 6-(benzyl-amino)-2(*S*)-[[1-(hydroxymethyl)propyl]amino]-9-isopropylpurine) or one of its pharmaceutically acceptable salts is present in said medication in an amount ranging from 100 mg to 5 g per unit, preferably from 100 mg to 2 g.

**14.** Use according to any one of claims 1 to 13, wherein said medication further comprises a pharmaceutically acceptable carrier.

**Patentansprüche**

**1.** Verwendung von 6-(Benzyl-amino)-2(*S*)-[[1-(hydroxymethyl)propyl]amino]-9-isopropylpurin) in einem enantiomerischen Überschuss von größer oder gleich 90 % oder zumindest eines seiner pharmazeutisch akzeptablen Salze zur Herstellung einer Medikaments zur Vorbeugung und/oder Behandlung chronischer neurologischer Krankheiten, die aus der Gruppe gewählt werden, zu der folgende Krankheiten gehören :

- neurodegenerative Krankheiten ;
- Demenzen ; und
- demyelinisierende Erkrankungen ;

und akute neurologische Erkrankungen, die aus der Gruppe gewählt werden, zu der folgende gehören:

- Epilepsie ;
- Status epilepticus ;
- Schlaganfall;
- Intracerebrale Blutungen ;
- cerebrale Hypoxien im Laufe von Herzstillständen;
- Schädel-Trauma; und
- neurologische Krankheiten, die eine fokale und/oder globale cerebrale Hypoxie hervorrufen.

**2.** Verwendung gemäß Anspruch 1, **dadurch gekennzeichnet, dass** besagte neurodegenerative Krankheiten innerhalb der Gruppe gewählt werden, zu der Krankheiten mit extrapyramidalem Syndrom, die amyotrophische Lateralsklerose und die Creutzfeldt-Jakob-Krankheit gehören.

**3.** Verwendung gemäß Anspruch 2, **dadurch gekennzeichnet, dass** besagte Krankheiten mit extrapyramidalem Syndrom in der Gruppe gewählt werden, zu der Parkinson-Krankheit, progressive supranukleare Lähmung, insbesondere die Steele-Richardson-Olszewski-Krankheit, Multisystematrophie, und striatonigrale Degeneration gehören.

**4.** Verwendung gemäß Anspruch 1, **dadurch gekennzeichnet, dass** besagte Demenzen innerhalb der Gruppe gewählt werden zur der Alzheimer, vaskuläre Demenzen, die Lewy-Körperchen-Krankheit, frontotemporale Demenzen, cortico-basale Degeneration, und Chorea Huntington gehören.

**5.** Verwendung gemäß Anspruch 1, **dadurch gekennzeichnet, dass** besagte demyelinisierende Erkrankungen innerhalb der Gruppe gewählt werden, zu der Multiple Sklerose, akute disseminierte Enzephalomyelitis, die Devic-Krankheit, und genetische Krankheiten mit Verletzung des Myelins gehören.

**6.** Verwendung gemäß Anspruch 1, **dadurch gekennzeichnet, dass** intracerebrale Blutungen in Verbindung mit der Verwendung eines thrombolytischen Mittels stehen.

**7.** Verwendung gemäß Anspruch 6, **dadurch gekennzeichnet, dass** das thrombolytische Mittel der gewebespezifische Plasmogenaktivator ist.

**8.** Verwendung gemäß einem beliebigen der Ansprüche 1 bis 7, **dadurch gekennzeichnet, dass** besagtes Medikament zudem mindestens ein anti-neurodegeneratives Mittel enthält, das aus der Gruppe gewählt wird, zu der Donepezil, Selegilin, Rivastigmin, Galantamin, Memantin, und Riluzol gehören.

**9.** Verwendung gemäß einem beliebigen der Ansprüche 1 bis 8, **dadurch gekennzeichnet, dass** besagtes Medikament zudem mindestens ein thrombolytisches Mittel enthält, das aus der Gruppe gewählt wird, zu welcher der gewebespezifische Plasmogenaktivator, Streptokinase, Urokinase, und Desmoteplase gehören.

**10.** Verwendung gemäß einem beliebigen der Ansprüche 1 bis 9, **dadurch gekennzeichnet, dass** besagtes Medikament zudem mindestens ein Mittel zur Thrombozytenaggregationshemmung enthält, das aus der Gruppe gewählt wird, zu der Aspirin, Ticlopidin, Clopidogrel, Persantin, Abciximab, und Flurbiprofen gehören.

**11.** Verwendung gemäß einem beliebigen der Ansprüche 1 bis 10, **dadurch gekennzeichnet, dass** besagtes Medikament oral, rektal, kutan, pulmonär, nasal, sublingual, parenteral, insbesonders intradermisch, subkutan, intramuskulär, intravenös, intraarteriell, intrarachidisch, intra-artikulär, intrapleural, intraperitoneal verabreicht werden kann.

**12.** Verwendung gemäß einem beliebigen der Ansprüche 1 bis 11, **dadurch gekennzeichnet, dass** besagtes Medikament innerhalb einer Gruppe gewählt wird, zu der Tabletten, Kapseln, Pillen, Sirup, Suspensionen, Lösungen, Pulver, Granulat, Emulsionen, Mikrosphären, injektierbare Lösungen, sublinguale Sprays und Hautpflaster gehören.

**13.** Verwendung gemäß einem beliebigen der Ansprüche 1 bis 12, **dadurch gekennzeichnet, dass** besagtes 6-(Benzylamino)-2(*S*)-[[1-(hydroxymethyl)propyl]amino]-9-isopropylpurine) oder eines seiner pharmazeutisch akzeptablen Salze in besagtem Medikament in einer Menge von 100 mg bis 5 g pro Einnahmeeinheit, vorzugsweise von 100 mg bis 2 g vorliegt.

**14.** Verwendung gemäß einem beliebigen der Ansprüche 1 bis 13, **dadurch gekennzeichnet, dass** besagtes Medikament zusätzlich einen pharmazeutisch akzeptablen Trägerstoff enthält.

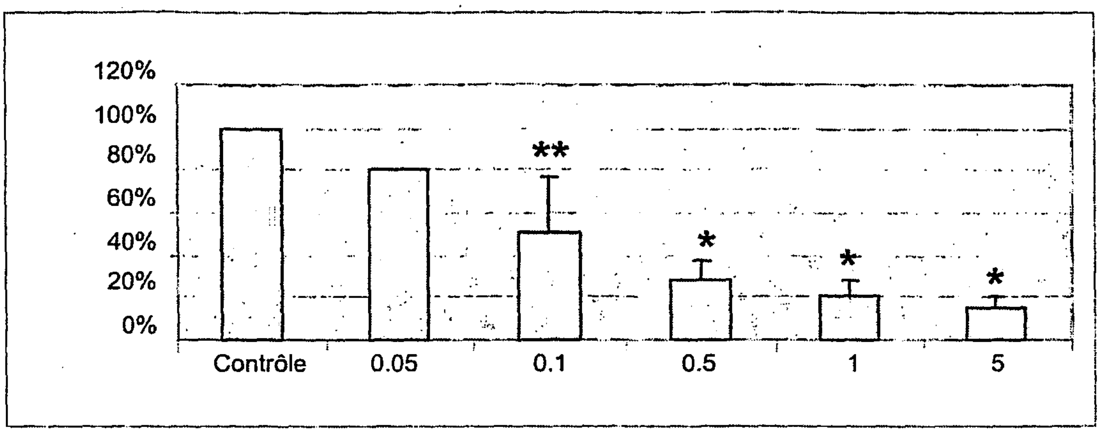


FIGURE 1

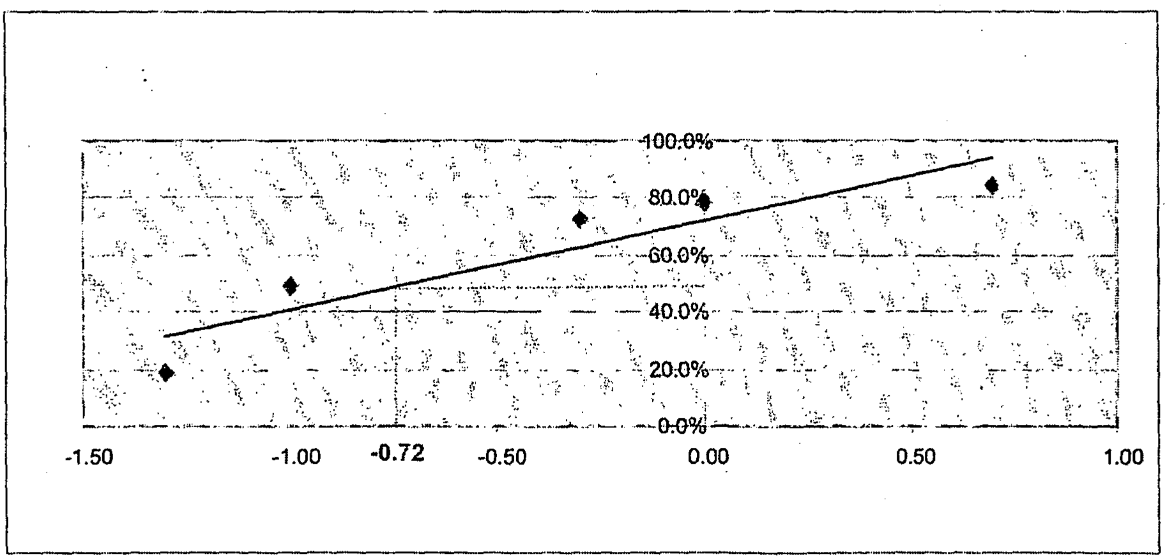


FIGURE 2

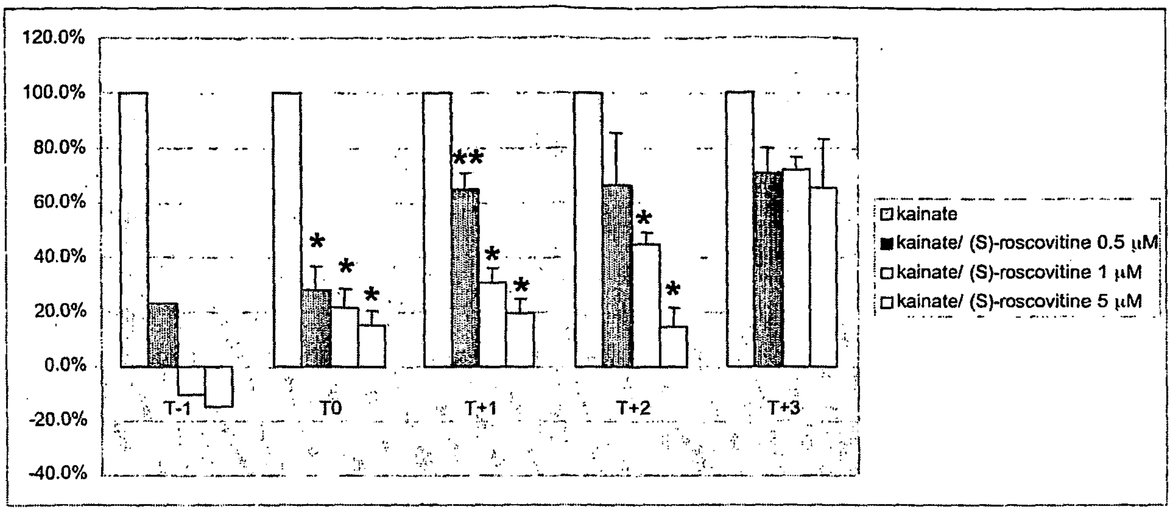


FIGURE 3

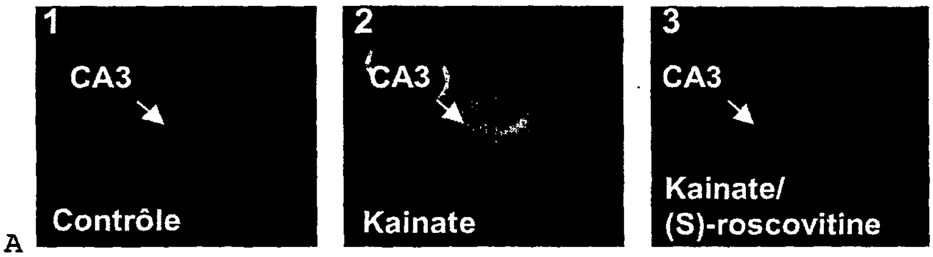


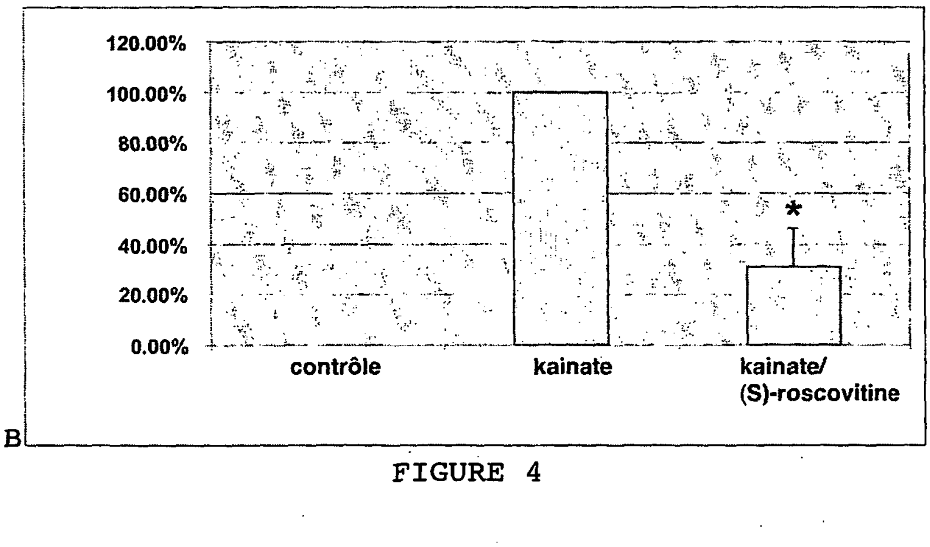


FIGURE 4

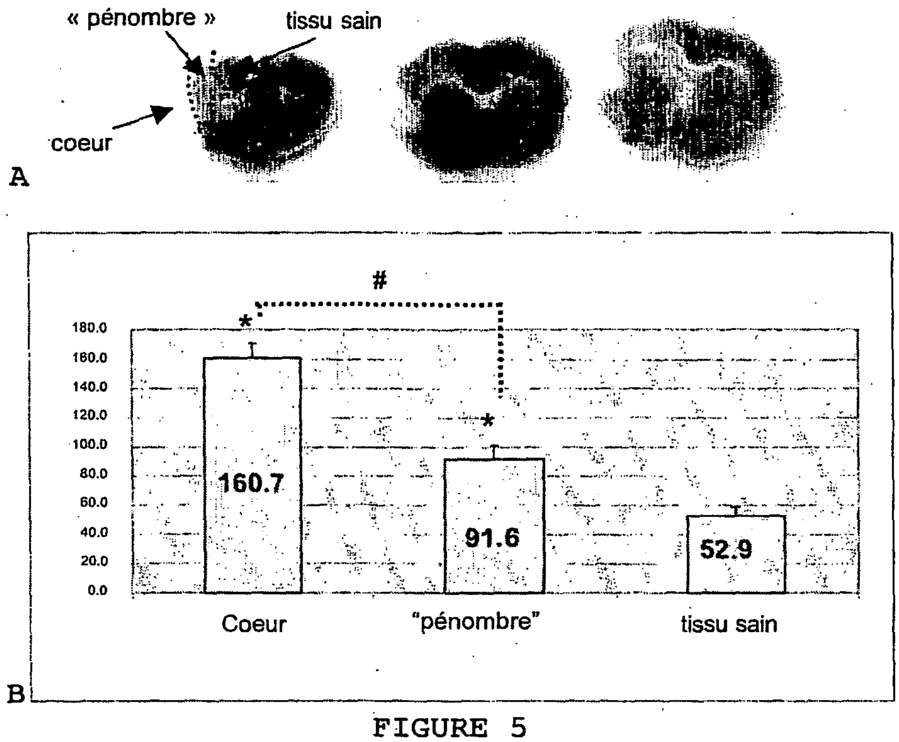


FIGURE 5

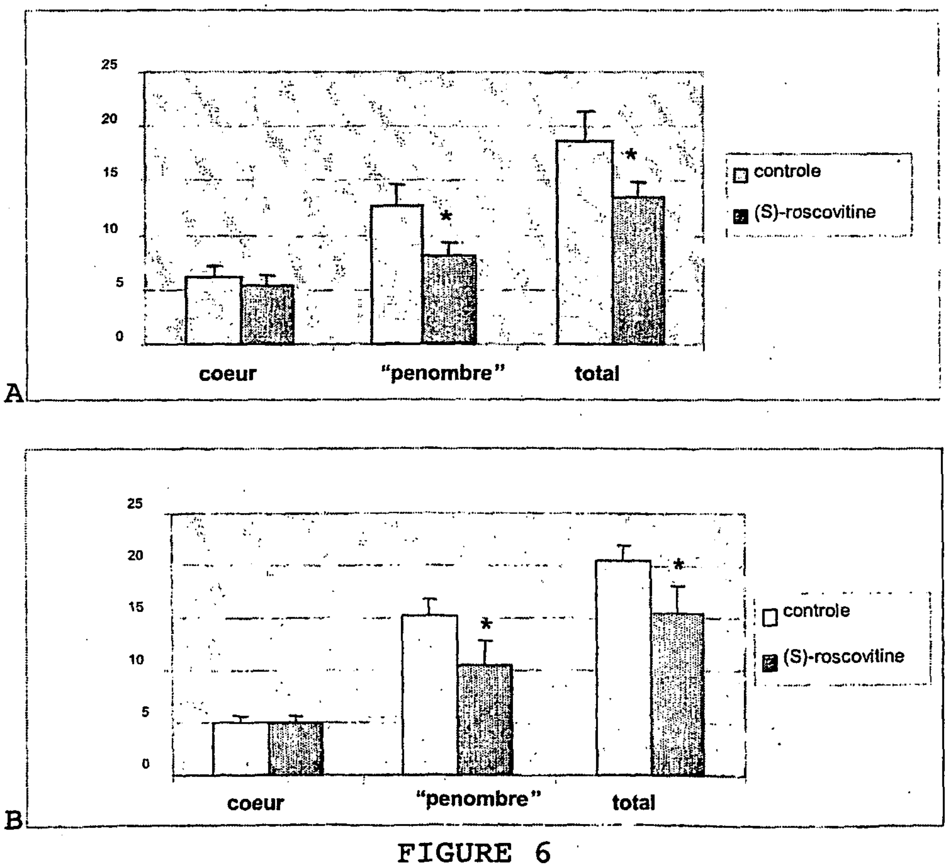


FIGURE 6

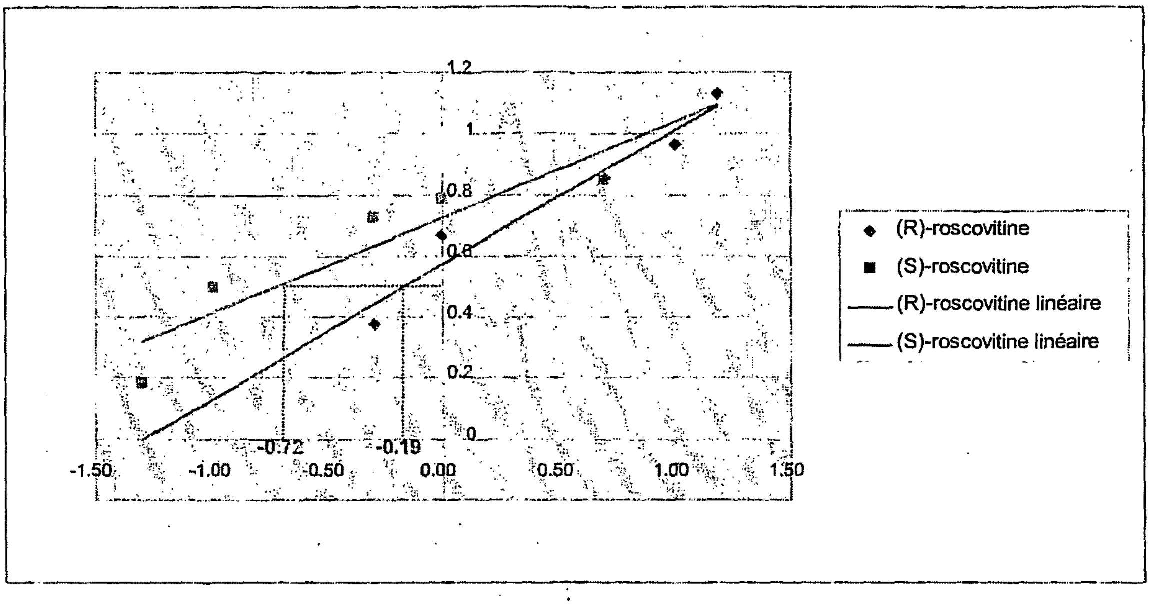
1.2
1
0.8
0.6
0.4
0.2
0
-1.50
-1.00
-0.72
-0.50
-0.19
0.00
0.50
1.00
1.50
(R)-roscovitine
(S)-roscovitine
(R)-roscovitine linéaire
(S)-roscovitine linéaire

FIGURE 7

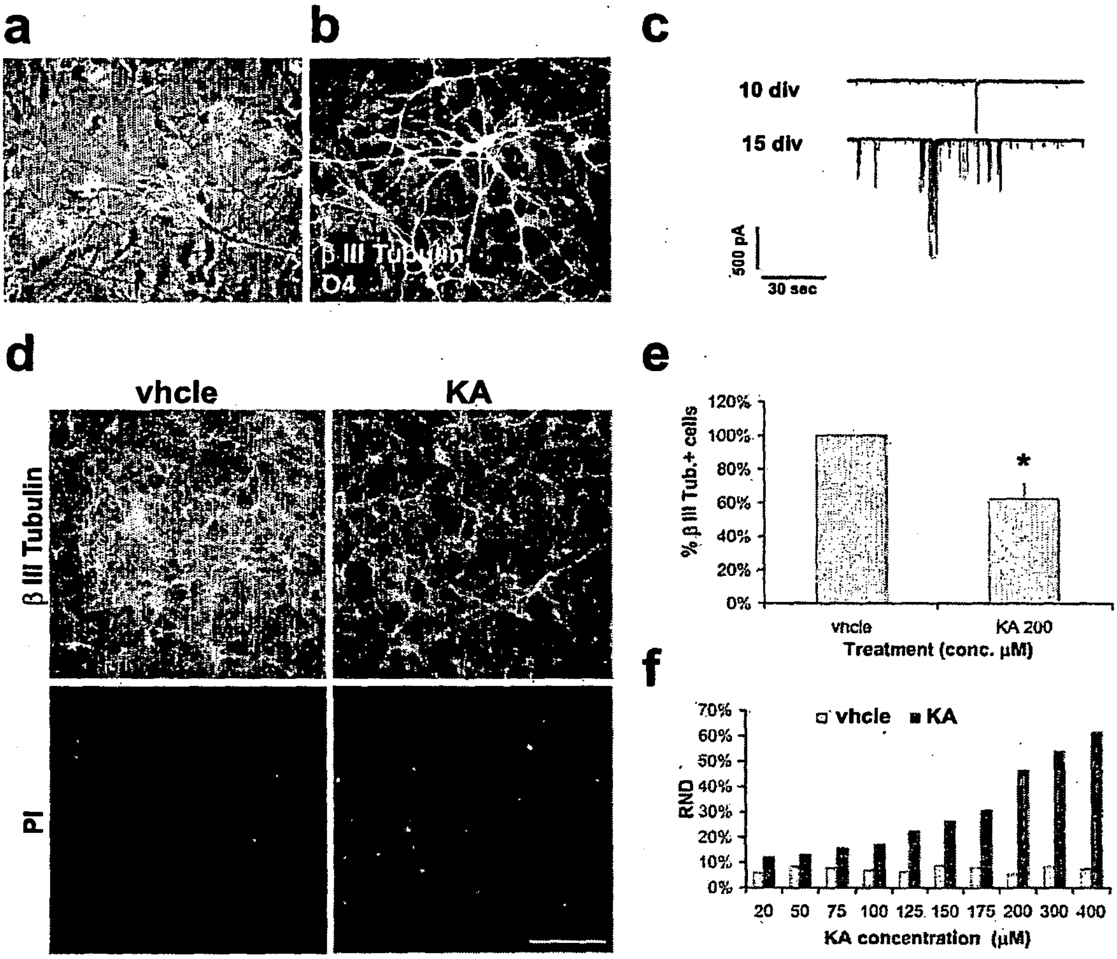
a
b
β III Tubulin
O4
c
10 div
15 div
500 pA
30 sec
d
vhcle
KA
β III Tubulin
PI
e
% β III Tub.+ cells
120%
100%
80%
60%
40%
20%
0%
*
vhcle
KA 200
Treatment (conc. μM)
f
vhcle
KA
RND
70%
60%
50%
40%
30%
20%
10%
0%
20 50 75 100 125 150 175 200 300 400
KA concentration (μM)

FIGURE 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0874847 A
- EP 0911634 A
- WO 9720842 A
- WO 0170231 A
- WO 2006021803 A


### Littérature non-brevet citée dans la description


- Excitotoxic cell death. **Olney JW ; Ishimaru MJ.** Cell death and diseases of the nervous system. Humana Press Inc, 1999, 197-219
- **Choi.** *Trends Neurosci,* 1988, vol. 11, 465-459
- **Coyle ; Puttfarcken.** *Science,* 1993, vol. 262, 689-695
- **Lipton ; Rosenberg.** *New Engl J Med,* 1994, vol. 330, 613-622
- **Vermeulen et al.** *Cell Prolif.,* 2003, vol. 36 (3), 131-49
- **De Azevedo et al.** *Eur. J. Biochem.,* 1997, vol. 243, 518-526
- **Bach et al.** *The Journal of Biochemical Chemistry,* vol. 280 (35), 31208-31219
- **Havlicek et al.** *J. Med. Chem,* 1997, vol. 40, 408
- **Wang et al.** *Tetrahefron : Asymmetry,* 2001, vol. 12, 2891
- **Adams ; Victor.** les maladies neurodégénératives. McGraw-Hill book company, 1985
- Remington's Pharmaceutical Sciences. Mack Publishing Co, 2000
- **Reisberg.** *N. Eng. J. Med,* 2003, vol. 348, 1333-1341
- **Medina et al.** *J. Neurophysiol.,* 1994, vol. 72, 456-465
- **Stoppini et al.** *J Neurosci Methods,* 1991, vol. 37, 173-182
- **Muller et al.** *Dev Brain Res,* 1993, vol. 71, 93-100
- **Buchs et al.** *Dev Brain Res,* 1993, vol. 71, 81-91
- **Tamura et al.** *J Cereb Blood Flow Metab,* 1981, vol. 1, 53-60
- **Guegan et al.** *Exp Neurol,* 1998, vol. 154, 371-380
- **Guegan et al.** *Mol Brain Res,* 1998, vol. 55, 133-140